# EUROPEAN PATENT APPLICATION

(11) **EP 4 746 439 A1**
(43) Date of publication of application: **20.05.2026**
(21) Application number: 24839427.2
(22) Date of filing: 17.06.2024
(51) Int. Cl.: H04N 25/76, A61B 1/04, A61B 1/045, H04N 25/677

(54) **IMAGING DEVICE, ELECTRONIC ENDOSCOPE SYSTEM, DATA PROCESSING METHOD, AND PROGRAM**

(30) Priority: 11.07.2023 JP 2023113557
(71) Applicant: HOYA CORPORATION, Shinjuku-ku Tokyo 160-8347 (JP)
(72) Inventor: TACHIBANA Toshio, Tokyo 160-8347 (JP)
(74) Representative: Lucke, Andreas
(86) International application number: PCT/JP2024/021888
(87) International publication number: WO 2025/013531

(57) **Abstract**

According to an aspect of the present invention, provided is an imaging device including: an image sensor configured such that adjacent pixel columns share a readout line; a readout unit that sequentially reads out data of G pixels in the adjacent pixel columns via a common terminal, and sequentially reads out data of R pixels and data of B pixels in the adjacent pixel columns via the common terminal; an acquisition unit that acquires, for light-shielded pixel regions set in a plurality of pixel rows in the image sensor, reference data corresponding to each terminal based on data of pixels read out via the same terminal; and a correction unit that corrects data of each pixel in an effective pixel region by subtracting corresponding reference data from data of pixels read out via each terminal for the effective pixel region excluding the light-shielded pixel regions in the image sensor.

## Description

### Technical Field

The present invention relates to an imaging device configured to image biological tissue and an electronic endoscope system including an electronic endoscope having the imaging device at a distal end.

### Background Art

In a medical device field, there is known an electronic endoscope system capable of generating an image suitable for diagnosing a lesion hidden in a body cavity, by illuminating biological tissue in the body cavity and imaging the illuminated biological tissue in the body cavity as an object.

In a case of imaging biological tissue, when there are defective pixels which are generated during the manufacturing process and fail to output image data correctly in an image sensor used for imaging and when there is an abnormality in a transmission path within the image sensor, there is a possibility that vertical line-shaped defects occur on the captured image due to these factors. Such a vertical line-shaped defect is called fixed pattern noise (FPN). Since occurrence of the FPN is not preferable in terms of image quality, various methods for performing correction for reducing the FPN have been proposed.

For example, the specification of WO 2022/064609 A describes an imaging device including: a memory that stores, as a correction reference value, a gain that exceeds the gain obtained by a CMOS image sensor and at which linear FPN begins to occur in a captured image; and an image processing unit that performs line correction for suppressing FPN on the captured image in a case where a gain at the time of providing a gain exceeding the gain obtained by the CMOS image sensor exceeds the correction reference value.

### Summary of Invention

### Technical Problem

By the way, in a case where a CMOS image sensor is mounted on the distal end of the endoscope, it is necessary to use a small image sensor, and it is also difficult to mount an analog circuit board for sufficiently reducing FPN. Furthermore, in a case where the image sensor is used in the endoscope, the luminance of the illumination light is limited. However, when the gain set in the subsequent image processing is increased, the level of the FPN also increases. Therefore, it is difficult to reduce the FPN in the image processing. Therefore, in a case where the CMOS image sensor is mounted on the endoscope, it may be difficult to obtain original image quality of the image sensor.

An object of the present invention is to more effectively reduce fixed pattern noise included in an image obtained from an image sensor than in the related art.

### Solution to Problem

According to a first aspect of the present disclosure, there is provided an imaging device including:
an image sensor in which a color filter of either R (red), G (green), or B (blue) is disposed in a predetermined pattern for each pixel of a plurality of pixels arranged in a matrix and which is configured such that adjacent pixel columns share a readout line;
a readout unit that reads out data of each pixel via a plurality of terminals through the readout lines of the image sensor, the readout unit sequentially reading out data of pixels (G pixels) corresponding to G color filters in the adjacent pixel columns via a common terminal, and sequentially reading out data of pixels (R pixels) corresponding to R color filters and data of pixels (B pixels) corresponding to B color filters in the adjacent pixel columns via the common terminal;
an acquisition unit that acquires, for light-shielded pixel regions set in a plurality of pixel rows in the image sensor, reference data corresponding to each terminal based on data of pixels read out via the same terminal; and
a correction unit that corrects data of each pixel in an effective pixel region by subtracting corresponding reference data from data of pixels read out via each terminal for the effective pixel region excluding the light-shielded pixel regions in the image sensor.

According to a second aspect of the present disclosure, there is provided an imaging device including:
an image sensor in which a color filter of either R (red), G (green), or B (blue) is disposed in a predetermined pattern for each pixel of a plurality of pixels arranged in a matrix and which is configured such that adjacent pixel columns share a readout line;
a readout unit that reads out data of each pixel via a plurality of terminals through the readout lines of the image sensor, the readout unit reading out the data of each pixel in the image sensor in either a first readout mode in which data of pixels (G pixels) corresponding to G color filters in the adjacent pixel columns are sequentially read out via a common terminal, and data of pixels (R pixels) corresponding to R color filters and data of pixels (B pixels) corresponding to B color filters in the adjacent pixel columns are sequentially read out via the common terminal, or
a second readout mode in which the data of the G pixels and the data of the R pixels in the adjacent pixel columns are sequentially read out via the common terminal and the data of the G pixels and the data of the B pixels in the adjacent pixel columns are sequentially read out via the common terminal;
an acquisition unit that acquires, for light-shielded pixel regions set in a plurality of pixel rows in the image sensor, reference data corresponding to each terminal based on data of pixels read out via each terminal;
a control unit that sets a readout mode when the readout unit reads out data of each pixel in the image sensor based on the reference data acquired by the acquisition unit in either the first readout mode or the second readout mode; and
a correction unit that corrects data of each pixel in an effective pixel region by subtracting corresponding reference data from data of pixels read out via each terminal in the readout mode set by the control unit for the effective pixel region excluding the light-shielded pixel regions in the image sensor.

The readout unit may include an analog-to-digital conversion unit including a plurality of analog-to-digital converters that convert, into digital data, data read out along pixel columns of the image sensor via a predetermined number of terminals among the plurality of terminals. In this case, each of the analog-to-digital converters is configured to sequentially process the data read out via the predetermined number of terminals.

According to a third aspect of the present disclosure, there is provided an electronic endoscope system including: the imaging device for imaging biological tissue; and a light source device that emits illumination light to the biological tissue.

The light source device is capable of selecting either white light or special light as the illumination light, and the control unit sets a readout mode when the readout unit reads out data of each pixel in the image sensor at a time of activation or at a timing when the illumination light is selected.

In a case where the reference data acquired in either the first readout mode or the second readout mode is less than a predetermined level, the control unit may set the mode to a readout mode when the readout unit reads out data of each pixel in the image sensor.

In a case where the reference data acquired in either the first readout mode or the second readout mode is equal to or greater than a predetermined level, the control unit may compare the reference data with reference data acquired in the other mode, and set the readout mode corresponding to smaller reference data to a readout mode when the readout unit reads out data of each pixel in the image sensor.

According to a fourth aspect of the present disclosure, there is provided a data processing method in a case where an object is imaged using an image sensor in which a color filter of either R (red), G (green), or B (blue) is disposed in a predetermined pattern for each pixel of a plurality of pixels arranged in a matrix and which is configured such that adjacent pixel columns share a readout line.

This data processing method includes:
a step of reading out data of each pixel in the image sensor in a first readout mode in which data of pixels (G pixels) corresponding to G color filters in the adjacent pixel columns are sequentially read out via a common terminal and data of pixels (R pixels) corresponding to R color filters and data of pixels (B pixels) corresponding to B color filters in the adjacent pixel columns are sequentially read out via the common terminal in a case where data of each pixel is read out via a plurality of terminals through the readout lines of the image sensor for light-shielded pixel regions set in a plurality of pixel rows in the image sensor, and acquiring reference data corresponding to each terminal based on the data of pixels read out via each terminal;
a step of reading out data of each pixel in the image sensor in a second readout mode in which the data of the G pixels and the data of the R pixels in the adjacent pixel columns are sequentially read out via the common terminal and the data of the G pixels and the data of the B pixels in the adjacent pixel columns are sequentially read out via the common terminal in a case where the data of each pixel is read out via the plurality of terminals through the readout lines of the image sensor for the light-shielded pixel regions, and acquiring reference data corresponding to each terminal based on the data of pixels read out via each terminal;
a step of setting a readout mode based on the reference data acquired in either the first readout mode or the second readout mode; and
a step of correcting data of each pixel in an effective pixel region by subtracting corresponding reference data from data of pixels read out via each terminal in the set readout mode for the effective pixel region excluding the light-shielded pixel regions in the image sensor.

According to a fifth aspect of the present disclosure, there is provided a program for causing a computer to execute a procedure in a case where an object is imaged using an image sensor in which a color filter of either R (red), G (green), or B (blue) is disposed in a predetermined pattern for each pixel of a plurality of pixels arranged in a matrix and which is configured such that adjacent pixel columns share a readout line, the procedure including:
a step of reading out data of each pixel in the image sensor in a first readout mode in which data of pixels (G pixels) corresponding to G color filters in the adjacent pixel columns are sequentially read out via a common terminal and data of pixels (R pixels) corresponding to R color filters and data of pixels (B pixels) corresponding to B color filters in the adjacent pixel columns are sequentially read out via the common terminal in a case where data of each pixel is read out via a plurality of terminals through the readout lines of the image sensor for light-shielded pixel regions set in a plurality of pixel rows in the image sensor, and acquiring reference data corresponding to each terminal based on the data of pixels read out via each terminal;
a step of reading out data of each pixel in the image sensor in a second readout mode in which the data of the G pixels and the data of the R pixels in the adjacent pixel columns are sequentially read out via the common terminal and the data of the G pixels and the data of the B pixels in the adjacent pixel columns are sequentially read out via the common terminal in a case where the data of each pixel is read out via the plurality of terminals through the readout lines of the image sensor for the light-shielded pixel regions, and acquiring reference data corresponding to each terminal based on the data of pixels read out via each terminal;
a step of setting a readout mode based on the reference data acquired in either the first readout mode or the second readout mode; and
a step of correcting data of each pixel in an effective pixel region by subtracting corresponding reference data from data of pixels read out via each terminal in the set readout mode for the effective pixel region excluding the light-shielded pixel regions in the image sensor.

### Advantageous Effects of Invention

In the imaging device according to the present disclosure, it is possible to more effectively reduce fixed pattern noise included in an image obtained from an image sensor than in the related art.

### Brief Description of Drawings

Fig. 1 is a block diagram illustrating an example of a configuration of an electronic endoscope system according to an embodiment.
Fig. 2 is a diagram illustrating a pixel array of a CMOS image sensor.
Fig. 3 is a block diagram illustrating an example of a configuration of a CMOS image sensor and a control unit in an electronic endoscope system according to the embodiment.
Fig. 4 is a diagram more specifically illustrating a pixel array, a switch unit, and an ADC unit in the CMOS image sensor of Fig. 3.
Fig. 5 is an example of a timing chart at the time of normal readout of pixel data in the CMOS image sensor of Fig. 3.
Fig. 6 is an example of a timing chart at the time of shift readout of pixel data in the CMOS image sensor of Fig. 3.
Fig. 7 is a diagram illustrating an example of readout of pixel data in the CMOS image sensor of Fig. 3.
Fig. 8 is a diagram illustrating an example of readout of pixel data in the CMOS image sensor of Fig. 3.
Fig. 9 is a flowchart illustrating a readout mode setting process in an electronic endoscope system according to the embodiment.

### Description of Embodiments

Hereinafter, an electronic endoscope system of an imaging system according to an embodiment of the present invention will be described in detail with reference to the drawings.

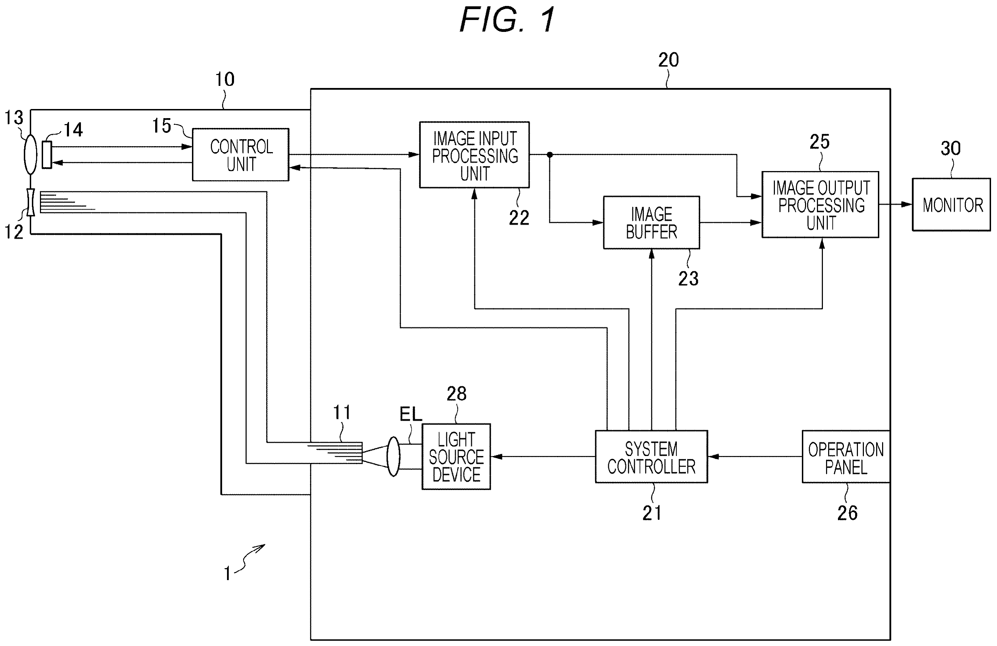

Fig. 1 is a block diagram illustrating an example of a configuration of an electronic endoscope system 1 according to the embodiment. The electronic endoscope system 1 is a system specialized for medical use, and is used to observe and diagnose biological tissue inside a body cavity.

As illustrated in Fig. 1, the electronic endoscope system 1 according to the embodiment includes an electronic scope (endoscope) 10, a processor 20 incorporating a light source device, and a monitor 30. The electronic scope 10 and the processor 20 are connected by a connector, and the processor 20 is connected to the monitor 30. A portion of the electronic endoscope system 1, excluding a light source device 28, is an example of an imaging device.

The processor 20 includes a system controller 21, an image input processing unit 22, an image buffer 23, an image output processing unit 25, an operation panel 26, and a light source device 28.

The system controller 21 executes various programs and integrally controls the entire electronic endoscope system 1. The system controller 21 executes operations of the electronic endoscope system 1 and changes parameters for the operations in accordance with an operator's (observer's) instruction input to the operation panel 26. The system controller 21 supplies clock pulses for adjusting an operation timing of each unit to the corresponding circuits in the electronic endoscope system 1.

The light source device 28 generates emission light EL (illumination light) for illuminating an object. The emission light EL may be white light, pseudo-white light, or special light in a specific wavelength band. In the embodiment, the light source device 28 can switch the emission light EL between white light and special light (illumination light can be selected) in accordance with an instruction of the system controller 21.

The emission light EL of the light source device 28 is condensed onto an incident end face of a light carrying bundle (LCB) 11 by a condenser lens, and enters the LCB 11. The type of light source of the light source device 28 is not limited, and examples of the light source include, for example, an LED, a laser diode, and a high-luminance lamp (for example, a xenon lamp, a metal halide lamp, a mercury lamp, or a halogen lamp).

Note that in the electronic endoscope system 1 illustrated in Fig. 1, the light source device 28 is incorporated in the processor 20, but the present invention is not limited thereto. The light source device 28 may be incorporated in the electronic scope 10, or may be provided separately from the electronic scope 10 and the processor 20 and connected to each other by wire.

The emission light EL that enters the LCB 11 propagates through the LCB 11. The emission light EL propagating through the LCB 11 is emitted from an emission end face of the LCB 11 disposed at a distal end of the electronic scope 10, and emitted to the object via a light distribution lens 12. Return light from the object illuminated with the emission light EL from the light distribution lens 12 forms an optical image on a light reception surface of a CMOS image sensor 14 via an objective lens 13.

The CMOS image sensor 14 is an image sensor configured to image an object by a rolling shutter method. The CMOS image sensor 14 has a pixel array with a Bayer pattern pixel arrangement in which a color filter of either R (red), G (green), or B (blue) is disposed in a predetermined pattern for each of a plurality of pixels arranged in a matrix on the light reception surface. In the following description, the pixels corresponding to the R, G, and B color filters are respectively referred to as "R pixels", "G pixels", and "B pixels".

The CMOS image sensor 14 accumulates an optical image formed at each pixel of the pixel array as a charge corresponding to the amount of light, and generates and outputs R pixel, G pixel, and B pixel data. Note that a charge-coupled device (CCD) image sensor or another type of imaging device may be applied instead of the CMOS image sensor 14.

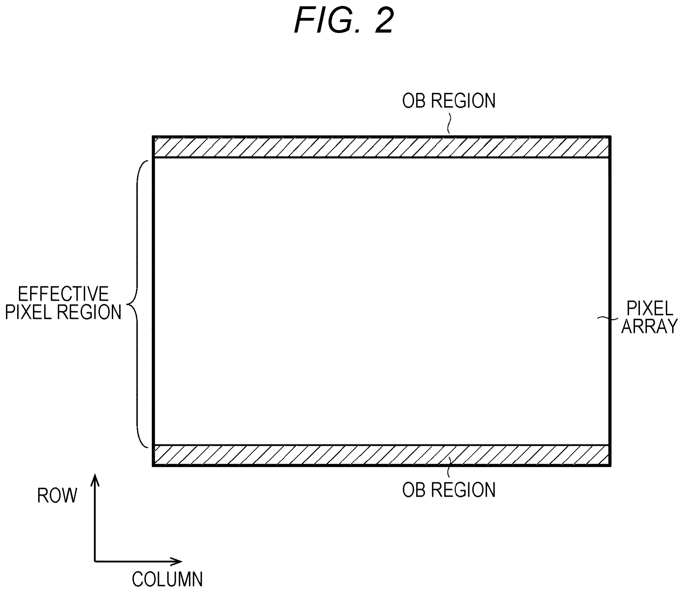

Fig. 2 illustrates a configuration of the pixel array of the CMOS image sensor 14.

As illustrated in Fig. 2, in the pixel array of the CMOS image sensor 14, a plurality of pixels are arranged in a matrix in pixel rows and pixel columns on the light reception surface. The pixel array includes an effective pixel region and an OB region (an example of a light-shielded pixel region). The effective pixel region is a region that is not shielded from light in the pixel array. Image data obtained based on the pixel data in the effective pixel region is sent to the image input processing unit 22.

The OB (optical black) region is a band-shaped light-shielded region along a horizontal direction (that is, a column direction) in the pixel array, is set in the upper end region and the lower end region of the pixel array, and includes a predetermined number of pixel rows. Although the number of pixel rows corresponding to the OB region is not limited, for example, several tens of rows are set in each of the upper end region and the lower end region. In the example illustrated in Fig. 2, the OB region is set in both the upper end region and the lower end region of the pixel array, but the present invention is not limited thereto, and may be set in either the upper end region or the lower end region. Note that the OB region may be set not only in the horizontal direction but also in a left end region and a right end region of the pixel array along a vertical direction in the pixel array.

As will be described later, pixel data obtained in the OB region is used for detecting FPN and correcting each pixel data in the effective pixel region.

Referring to Fig. 1 again, a control unit 15 provided in the electronic scope 10 drives and controls the CMOS image sensor 14 at a timing synchronized with the frame rate of the video processed on the processor 20 side in accordance with the clock pulse supplied from the system controller 21. An imaging signal obtained by imaging an object from the CMOS image sensor 14 is input to the control unit 15 at a predetermined frame period. The frame period (one frame period) is, for example, 1/120 second, 1/60 second, or 1/30 second.

The control unit 15 also detects fixed pattern noise (hereinafter referred to as "FPN") from the image data input from the CMOS image sensor 14, and corrects the image data so as to reduce the FPN. The control unit 15 outputs the corrected image data (captured image) to the image input processing unit 22 of the processor 20. The control unit 15 will be described in more detail later.

In the processor 20, the image input processing unit 22, the image buffer 23, and the image output processing unit 25 are provided to generate an image to be displayed on the monitor 30.

The image input processing unit 22 performs predetermined processing such as demosaic processing and matrix operations performed on frame-unit image data (captured images) sent from the control unit 15, and transmits the processed image data to the image buffer 23 and the image output processing unit 25.

The image buffer 23 is a memory (frame buffer) that buffers the captured images sent from the image input processing unit 22 on a frame basis. The image buffer 23 temporarily stores the captured images of past frames, such as frames one frame before and two frames before the current frame, in order to perform processing in the image output processing unit 25.

The image output processing unit 25 performs image processing such as filtering processing or edge enhancement processing based on the image data of the current frame output from the image input processing unit 22 and the image data of the past frame acquired from the image buffer 23 to generate screen data for monitor display, and converts the generated screen data for monitor display into a predetermined video format signal. The converted video format signal is output to the monitor 30. Thus, an image of the object is displayed on a display screen of the monitor 30.

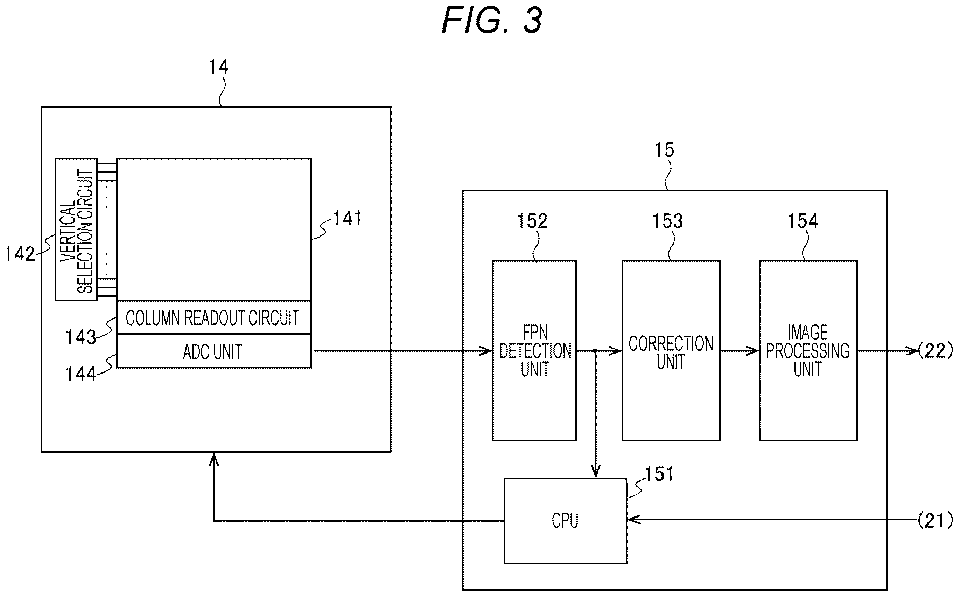

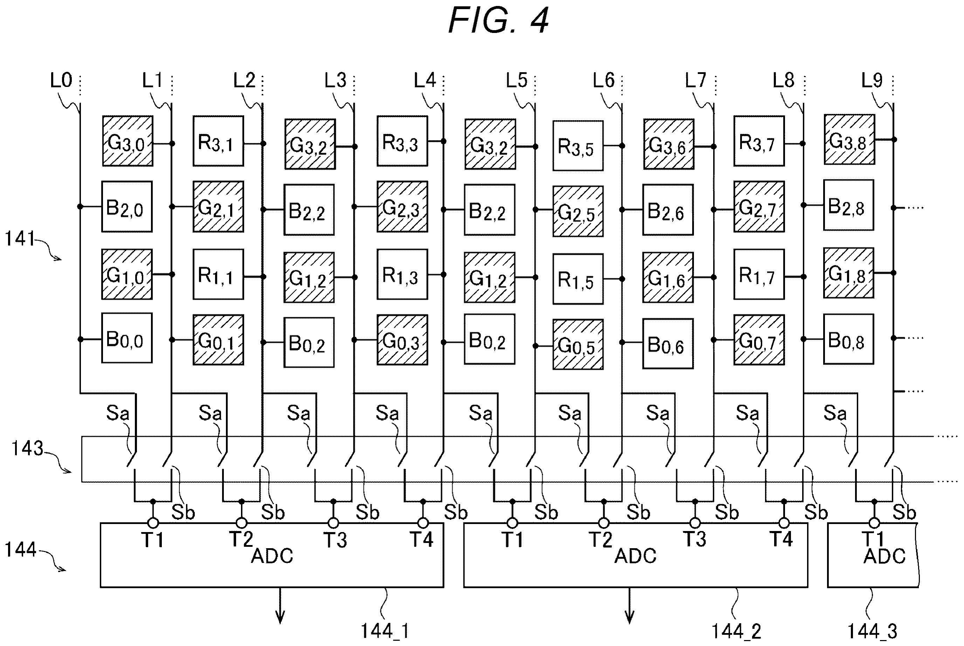

Next, the CMOS image sensor 14 and the control unit 15 will be described in more detail with reference to Figs. 3 and 4.

Fig. 3 is a block diagram illustrating an example of a configuration of the CMOS image sensor 14 and the control unit 15 in the electronic endoscope system 1.

As illustrated in Fig. 3, the CMOS image sensor 14 includes a pixel array 141, a vertical selection circuit 142, a column readout circuit 143, and an analog-to-digital converter (ADC) unit 144. Fig. 4 is a diagram more specifically illustrating the pixel array 141, the column readout circuit 143, and the ADC unit 144 in the CMOS image sensor 14 of Fig. 3.

Referring to Fig. 3, as illustrated in Fig. 2, the pixel array 141 has a plurality of pixels arranged in a matrix, and includes an effective pixel region and an OB region.

In the pixel array 141 illustrated in Fig. 4, a part of a plurality of pixels arranged in a matrix is illustrated. In the pixel array 141, a color filter of either R (red), G (green), or B (blue) is disposed for each pixel of a plurality of pixels arranged in a matrix in a Bayer pattern pixel arrangement. That is, G pixels indicated by hatching are disposed in a checkerboard pattern.

In the pixel array 141 of Fig. 4, B_{i,j}(i=0, 1,..., j=0, 1,...) represents a pixel (B pixel) on which a B (blue) color filter is disposed. G_{i,j}(i=0, 1,..., j=0, 1,...) represents a pixel (G pixel) on which a G (green) color filter is disposed. R_{i,j}(i=0, 1,..., j=0, 1,...) represents a pixel (R pixel) on which a R (red) color filter is disposed.

A plurality of readout lines Lo, L1,..., L9,... are provided corresponding to respective pixel column of the pixel array 141. The pixel array 141 is configured to share a readout line between adjacent pixel columns.

In the embodiment, as illustrated in Fig. 4, each readout line is wired to be shared by pixels that are diagonally disposed. For example, the diagonally disposed pixels G_{0,1}, G_{1,0}, G_{2,1}, and G_{3,0} share one readout line L1.

The vertical selection circuit 142 is a circuit that sequentially selects pixel rows in the pixel array 141. The column readout circuit 143 is a circuit that sequentially selects pixel columns in the pixel array 141. For example, the vertical selection circuit 142 sequentially scans pixel rows, and pixel data output from a pixel row to be scanned is input to the column readout circuit 143 through the corresponding readout line. In the example illustrated in Fig. 4, each of the pixels B_{0,0}, G_{0,1}, B_{0,2},... in the zero-th row is selected, and the data of each selected pixel is output to the corresponding readout line. Subsequently, each of the pixels G_{1,0}, R_{1,1}, G_{1,2},... in the first row is selected, and the data of each selected pixel is output to the corresponding readout line.

The configuration of the pixel may be known, and includes, for example, a photodiode that performs photoelectric conversion, a floating diffusion (FD), an amplification transistor, and a plurality of switches including a transfer switch that transfers charge generated in the photodiode to the FD.

The column readout circuit 143 and the ADC unit 144 are an example of a readout unit that reads out data of each pixel via input terminals T1 to T4 of the ADC through readout lines of the CMOS image sensor 14. The data of the G pixels in adjacent pixel columns are sequentially read out via a common terminal (input terminal of the common ADC), and the data of the R pixels and the B pixels in adjacent pixel columns are sequentially read out via the common terminal (input terminal of the common ADC).

The column readout circuit 143 includes a plurality of switches that operate corresponding to either a normal readout mode or a shift readout mode to be described later. The normal readout mode is a mode for reading out each pixel of the pixel array 141 in accordance with the arrangement of the pixel columns, and is an example of a second readout mode. The shift readout mode is a mode for shifting each pixel of the pixel array 141 in the pixel column direction in accordance with the pixel row and reading each pixel, and is an example of the first readout mode.

In the example illustrated in Fig. 4, the column readout circuit 143 includes a plurality of switch pairs when the switches Sa and Sb form one switch pair. In each switch pair, one end of each of the switch Sa and the switch Sb is connected to an adjacent readout line, and the other end is a common output end (input terminal of the ADC). For example, focusing on the adjacent readout lines L0 and L1, one end of the switch Sa of the switch pair is connected to the readout line Lo, one end of the switch Sb is connected to the readout line L1, and the other ends of the switches Sa and Sb are common output ends (input terminal T1).

The ADC unit 144 converts, into digital data, data read out via the terminals provided at predetermined intervals along the pixel columns of the pixel array 141 (four input terminals T1 to T4 in the example of Fig. 4).

Specifically, the ADC unit 144 includes a plurality of ADCs 144_1, 144_2,..., and each ADC includes four input terminals T1 to T4. Each ADC is configured to sequentially process the data read out via four input terminals T1 to T4. The input terminals T1 to T4 of each ADC are connected to the output ends of four switch pairs provided in the column direction. That is, four adjacent pixel columns share one ADC. The number of pixel columns sharing one ADC is not limited to four, but the readout speed can be increased by sharing one ADC among a plurality of pixel columns. In the example illustrated in Fig. 4, the ADC unit 144 sequentially converts the pixel data input via each input terminal in each ADC into digital data with a time difference and reads out the digital data in such a manner that the pixel data input to the input terminal T1 of the ADCs 144_1, 144_2,... is acquired, subjected to A/D conversion, and read out as digital data, and sequentially the pixel data input to the input terminal T2 of the ADCs 144_1, 144_2,... is acquired, subjected to A/D conversion, and read out as digital data.

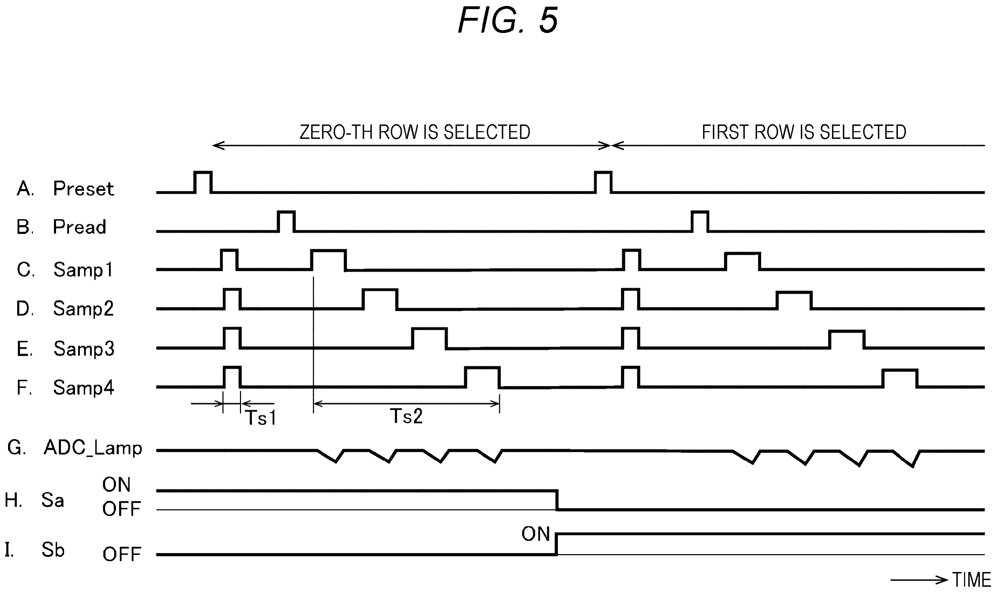

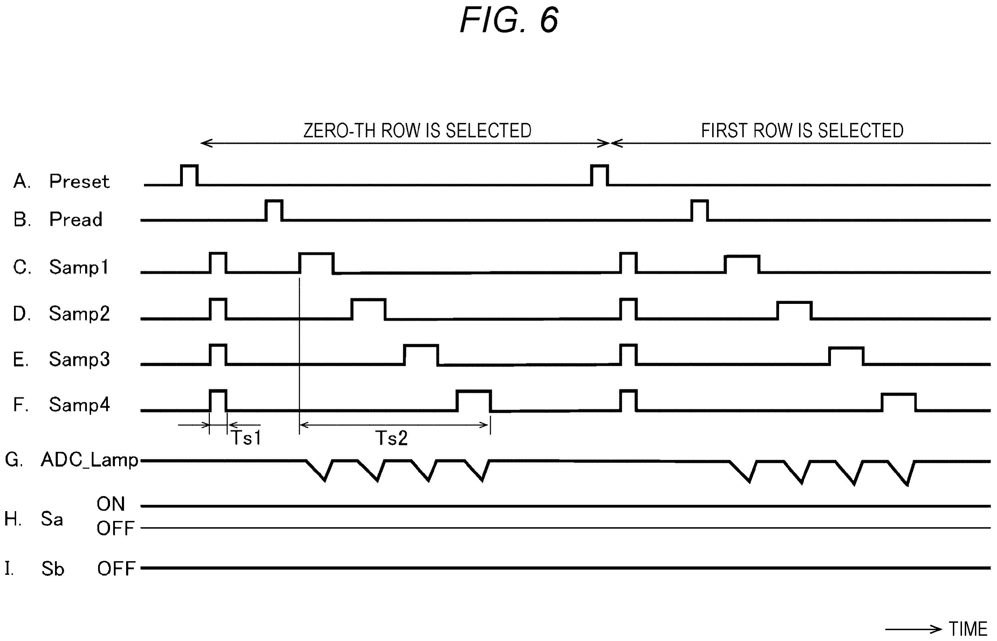

Next, the readout operations in the shift readout and normal readout will be described with reference to Figs. 5 and 6.

Fig. 5 is an example of a timing chart at the time of the normal readout of the pixel data in the CMOS image sensor 14 of Fig. 3. Fig. 6 is an example of a timing chart at the time of the shift readout of the pixel data in the CMOS image sensor 14 of Fig. 3. In each of Figs. 5 and 6, A. denotes a reset pulse P_{RESET}, B. denotes a readout pulse P_{READ}, C. to F. denote sampling (analog inputs) Samp1 to Samp4 input to the input terminals T1 to T4 of each ADC of the ADC unit 144, G. denotes an ADC analog ramp ADC_Lamp, H. denotes a state (ON or OFF) of the switch Sa, and I. denotes a state (ON or OFF) of the switch Sb.

Figs. 5 and 6 illustrate the data readout of the pixels in the zero-th row and the first row. When the pixel row is selected, the reset pulse P_{RESET} is transmitted to each pixel, and the FD of each pixel is reset (The FD becomes a reset-level potential).

Referring to Figs. 5 and 6, the control of the switches Sa and Sb differs depending on the selection result of either the shift readout or the normal readout.

In the normal readout illustrated in Fig. 5, the switch Sa is turned on and the switch Sb is turned off when the zero-th row is selected, and the switch Sa is turned off and the switch Sb is turned on when the first row is selected. Although the second and subsequent rows are not illustrated in Fig. 5, the state of each switch is switched every time a new row is selected.

On the other hand, in the shift readout illustrated in Fig. 6, when the zero-th row or the first row is selected, the switch Sa is turned on and the switch Sb is turned off. Although the second and subsequent rows are not illustrated in Fig. 6, even in a case where a new row is selected, the switch Sa is always turned on and the switch Sb is always turned off.

When a row is selected, the input terminals T1 to T4 of each ADC of the ADC unit 144 are connected to any one of the readout lines in accordance with the state of the switch.

When the zero-th row is selected, data (potential) corresponding to the reset level of the FD of each pixel in the zero-th row is output to the corresponding readout line and sampled via the input terminal of the ADC connected to the readout line (period Ts1). Next, the charge accumulated in the photodiode of each pixel is transferred to the FD in synchronization with the readout pulse P_{READ}, and is output to the readout line. The data (potential) output to the readout line is sampled via the input terminals T1 to T4 of each ADC with a time difference (period Ts2). Each ADC subtracts the reset-level data input in the period Ts1 from the data input in the period Ts2 (correlated double sampling (CDS) processing), and converts the data into a digital value. In the period Ts2, since processing is performed with a time difference for each input terminal, the processing load in each ADC is reduced.

Next, in a case where the first row is selected, the states of the switches Sa and Sb change, but the operation of each unit is the same as that in a case where the zero-th row is selected.

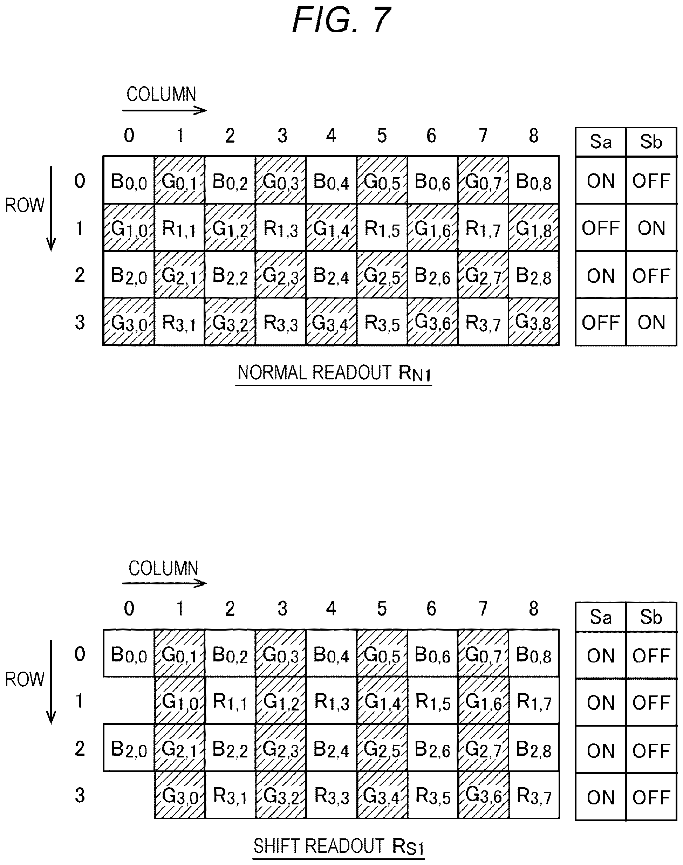

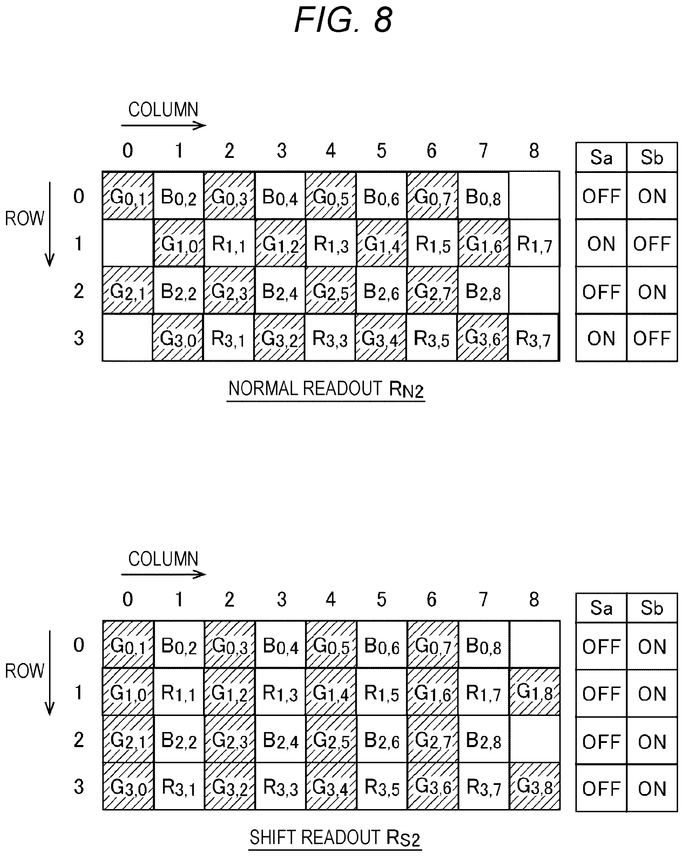

Next, with reference to Figs. 7 and 8, the normal readout and the shift readout in accordance with the control of the column readout circuit 143 will be described.

In each of Figs. 7 and 8, for a plurality of pixels of the pixel array 141 illustrated in Fig. 4, switch control when the zero-th to third rows are sequentially selected and scanned and a data arrangement of pixels read out when each row is selected are illustrated in association with each other. In Figs. 7 and 8, unlike the actual arrangement of pixels in the pixel array 141, the same column indicates pixels corresponding to data input via the same ADC input terminal. That is, "column" in Figs. 7 and 8 means a column of the input terminal of each ADC of the ADC unit 144, unlike the column in the pixel array of the pixel array 141.

Note that in Figs. 7 and 8, only the zero-th to third rows are illustrated, but the same applies to the fourth and subsequent rows.

Referring to Fig. 7, in normal readout R_{N1}, the state of each switch pair is switched each time a pixel row is selected. The switch Sa is turned on and the switch Sb is turned off when the zero-th row is selected, and the switch Sa is turned off and the switch Sb is turned on when the first row is selected. That is, the switch control in the normal readout R_{N1} corresponds to the flowchart of Fig. 5. In the normal readout R_{N1}, the G pixels after the readout are disposed in a checkerboard pattern.

In shift readout R_{S1}, the switch Sa is always turned on and the switch Sb is always turned off regardless of the selection of the pixel row. That is, the switch control in the shift readout R_{S1} corresponds to the flowchart of Fig. 6. In the shift readout R_{S1}, the data of the pixels connected to the odd-numbered rows (first row, third row,...) in the pixel array 141 are read out after being shifted one column to the right. As a result, the data of the G pixels disposed diagonally with respect to the common readout line are read out via the common terminal, and the data of the B pixels and the R pixels disposed diagonally with respect to the common readout line in the pixel array 141 are read via the common terminal.

Fig. 8 illustrates a case where the normal readout and the shift readout are performed by switch control different from that in Fig. 7.

Referring to Fig. 8, in normal readout R_{N2}, similarly to the normal readout R_{N1}, the state of each switch pair is switched every time a pixel row is selected, but the ON/OFF order for the switch pairs differs from that in the normal readout R_{N1}. The switch Sa is turned off and the switch Sb is turned on when the zero-th row is selected, and the switch Sa is turned on and the switch Sb is turned off when the first row is selected. In the normal readout R_{N2}, similarly to the normal readout R_{N1}, the G pixels after the readout are disposed in a checkerboard pattern.

In shift readout R_{S2}, the switch Sa is always turned off and the switch Sb is always turned on regardless of the selection of the pixel row. At this time, the data of the pixels connected to the even-numbered rows (zero-th row, second row,...) are read out after being shifted one column to the left. As a result, the data of the G pixels disposed diagonally with respect to the common readout line are read out via the common terminal, and the data of the B pixels and the R pixels disposed diagonally with respect to the common readout line are read out via the common terminal.

The description returns to Fig. 3.

Referring to Fig. 3, the control unit 15 includes a CPU 151, an FPN detection unit 152, a correction unit 153, and an image processing unit 154. By executing a predetermined program, the CPU 151 controls the entire operation of the control unit 15 and controls the vertical selection circuit 142 and the column readout circuit 143 of the CMOS image sensor 14 under the instruction of the system controller 21. Note that, in the embodiment, the processing of the control unit 15 may be performed by a device such as a digital signal processor (DSP), an ASIC, or a field programmable gate array (FPGA) instead of the CPU.

Based on the data of the pixels sequentially read out by the ADC unit 144, the FPN detection unit 152 detects fixed pattern noise (FPN; an example of the reference data) of the CMOS image sensor 14.

Specifically, the FPN detection unit 152 acquires, for the OB region (see Fig. 2) set in a plurality of pixel rows of the pixel array 141, reference data corresponding to each terminal based on pixel data read out via the same input terminal of each ADC. As described above, since the OB region is a band-shaped (predetermined row) light-shielding region along the horizontal direction (that is, the column direction), the data read out from the pixels in the OB region is data in which FPN is superimposed on the black level. The FPN detection unit 152 averages the data of the pixels in this predetermined row to detect the FPN. Since the FPN is a vertical line-shaped defect appearing on the captured image, the FPN is detected for each pixel column.

The correction unit 153 corrects the data of each pixel in the effective pixel region by subtracting the corresponding reference data (FPN detected by the FPN detection unit 152) from the pixel data read out via each input terminal of each ADC for the effective pixel region excluding the OB region of the pixel array 141. Since the FPN is detected for each pixel column as described above, the correction unit 153 subtracts the FPN detected corresponding to the pixel column from data of each pixel in a specific pixel column, and the data of each pixel is corrected.

Detection of FPN by the FPN detection unit 152 and correction by the correction unit 153 are performed on pixel data read out by the CMOS image sensor 14. Here, in a case where data of each pixel is read out through the shift readout (R_{S1} in Fig. 7 or R_{S2} in Fig. 8), data of the G pixels are read out via the common terminal. That is, as illustrated in Fig. 4, since the data of the diagonally disposed G pixels (for example, G_{0,1}, G_{1,0}, G_{2,1}, and G_{3,0}) are read out through the common readout line (for example, L1) and taken into the common input terminal, FPN caused by the same path is superimposed on the readout data. Therefore, since the number of samples at the time of detecting (calculating) FPN is doubled as compared with the normal readout, the FPN can be detected with high accuracy for the G pixel, and thus, the accuracy of correction for the data of the G pixel is improved. In general, since the luminance of the captured image obtained by the white or pseudo-white emission light EL has a high contribution of the G color, a good captured image can be obtained by the correction performed by the correction unit 153.

In the example illustrated in Fig. 4, the ADC unit 144 performs processing of sequentially converting pixel data (potential) input via each input terminal in each ADC into digital data with a time difference and reading out the data. For example, in the ADC 144_1, the pixel data is processed with a time difference in the order of the input terminals T1, T2, T3, and T4, and thus an offset corresponding to the time difference is superimposed on the data input at each input terminal. Therefore, conventionally, the FPN tends to increase in the order of the input terminals T1, T2, T3, and T4 even in a case where data of pixels with the same level is input. The difference in offset due to the input terminal can be reduced by taking the data of the G pixel through the common input terminal as described above.

The image processing unit 154 generates image data (captured image) including pixel data based on the original pixel array (Bayer pattern pixel arrangement) based on the pixel data corrected by the correction unit 153, and transmits the image data to the image input processing unit 22 on a frame basis. For example, in a case where the shift readout is selected, as illustrated in Figs. 7 and 8, the data of the G pixels are read out via the common input terminal, but are returned to the data of the Bayer pattern pixel arrangement (that is, data based on the array of the pixel array 141) by the image processing unit 154.

As described above, the electronic endoscope system 1 according to the embodiment executes shift readout in which the data of the G pixels are sequentially read out via the common terminal from the pixel array of the Bayer pattern pixel arrangement of the CMOS image sensor 14, and the data of the R pixels and the B pixels in the adjacent pixel columns are sequentially read out via the common terminal. Moreover, the electronic endoscope system 1 acquires FPN corresponding to each terminal based on the pixel data read out via the same terminal for the OB region of the CMOS image sensor 14, and corrects the data of each pixel in the effective pixel region by subtracting the corresponding FPN from the pixel data read out via each input terminal of the ADC for the effective pixel region. In the shift readout, since the data of the G pixels are taken into the common input terminal of the ADC, it is possible to detect the FPN with high accuracy for the G pixel. Therefore, the accuracy of correction for the data of the G pixels is improved. For example, in the case of white or pseudo-white emission light having a high contribution of the G color in luminance, a good captured image (that is, the image in which the fixed pattern noise is effectively reduced) can be obtained by correction.

Note that, for example, in a case where the illumination light is special light having high sensitivity to the B color, even when the illumination light is white, depending on the hue, it may be inappropriate to aggregate the G pixel data as data of the same column and read out the aggregated data. That is, in the embodiment, the normal readout may be selected instead of the shift readout in accordance with the illumination light emitted to the object.

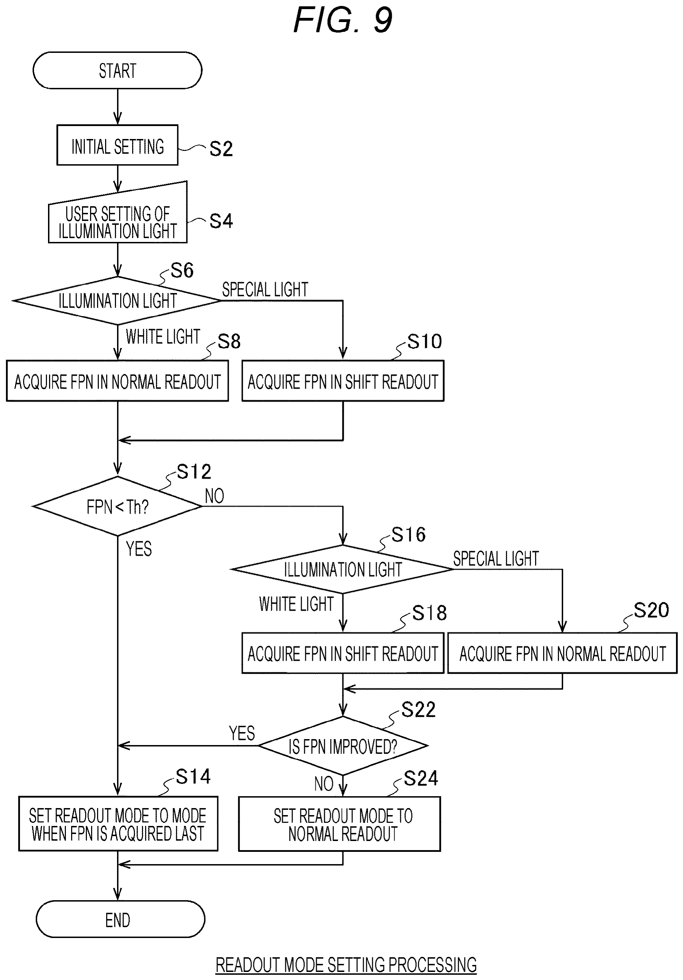

There is an individual difference in the manner in which FPN occurs in the CMOS image sensor 14, and the offset that occurs for each input terminal of each ADC is not limited to increasing in the order of input terminals T1, T2, T3, and T4, for example, a large offset may be generated only in two or three specific terminals. In the embodiment, it is also preferable to set the readout mode by comparing the FPNs acquired in both the shift readout mode and the normal readout mode. Hereinafter, an example of this readout mode setting process will be described with reference to a flowchart of Fig. 9.

The readout mode setting processing illustrated in Fig. 9 is processing mainly executed by the control unit 15 and the system controller 21 of the electronic endoscope system 1 in cooperation, the processing being executed immediately after the system is activated. Note that the readout mode setting processing may be performed at a timing when the illumination light is switched (the illumination light is selected) by the operator at the time of the system operation.

First, when an initial setting is completed (step S2), the system controller 21 sets illumination light based on a user setting of the illumination light input from the operation panel 26 (step S4). In a case where the set illumination light is white light (step S6), the system controller 21 instructs the control unit 15 to acquire FPN of the OB region through normal readout (step S8). In a case where the illumination light set in step S4 is special light (step S6), the system controller 21 instructs the control unit 15 to acquire FPN of the OB region through shift readout (step S10).

In a case where the FPN acquired in step S8 or S10 is smaller than a predetermined threshold Th (step S12: YES), the system controller 21 sets the readout mode to a readout mode when the FPN is acquired last (that is, when the FPN is acquired in step S8 or S10) (step S14). That is, in a case where FPN acquired in one of the shift readout mode and the normal readout mode is less than a threshold Th (an example of a predetermined level), the system controller 21 sets the readout mode to a mode in which the FPN is less than the predetermined level.

Note that the comparison between the FPN acquired in step S8 or S10 and the predetermined threshold Th may be performed based on the average value of the FPN acquired for each pixel column or based on the maximum value of the FPN for each pixel column.

On the other hand, in a case where the FPN acquired in step S8 or S10 is equal to or greater than the predetermined threshold Th (step S12: NO), the system controller 21 acquires FPN in a readout mode opposite to that in steps S6 to S10. That is, in a case where the illumination light set in step S4 is white light (step S16), the system controller 21 instructs the control unit 15 to acquire FPN of the OB region through shift readout (step S18). In a case where the illumination light set in step S4 is special light (step S16), the system controller 21 instructs the control unit 15 to acquire FPN of the OB region through normal readout (step S20).

In a case where the FPN acquired in step S18 or S20 is smaller than the FPN acquired in step S8 or S10 (that is, in a case where the FPN is improved) (step S22: YES), the system controller 21 sets the readout mode to a readout mode when the FPN is acquired last (that is, when the FPN is acquired in step S18 or S20) (step S14). That is, in a case where the FPN acquired in one of the shift readout mode and the normal readout mode in step S12 is equal to or greater than the threshold Th, the system controller 21 compares the FPN with the FPN acquired in the other mode, and sets the readout mode to a readout mode corresponding to the smaller FPN. Thus, it is possible to set the mode to a mode in which FPN is better (smaller) in the shift readout or the normal readout.

In a case where the FPN is not improved (step S22: NO), the system controller 21 sets the normal readout (step S24).

In the readout mode setting processing illustrated in Fig. 9, the readout mode in which FPN becomes low level is set based on the FPN acquired in each of the shift readout and normal readout. Therefore, the optimum readout mode can be set in accordance with the type of illumination light, the individual difference of the image sensor, and the like. Note that unlike Fig. 9, FPN may be acquired respectively in the shift readout and the normal readout in steps S8 and S10. FPN may be acquired respectively in the normal readout and the shift readout in steps S18 and S20. In any case, it is possible to set the mode to a mode in which FPN is better in the shift readout or the normal readout.

When the readout mode is set by the readout mode setting processing, the system controller 21 instructs the CPU 151 to perform the operation of the control unit 15 in accordance with the set readout mode. In accordance with this instruction, the control unit 15 subtracts the FPN (reference data) acquired by the readout mode setting processing from the pixel data read out via each input terminal of each ADC for the effective pixel region excluding the OB region of the pixel array 141, and corrects the data of each pixel in the effective pixel region.

Fig. 4 illustrates the pixel array in which each readout line is wired to be shared by pixels that are diagonally disposed, but the present invention is not limited thereto. It is also possible to configure such that pixels adjacent to each other in the horizontal direction or the vertical direction share a readout line.

In the pixel array 141 illustrated in Fig. 4, a plurality of pixels are configured in a Bayer pattern pixel arrangement, but the arrangement pattern of the color filters is not limited thereto, and may be any pattern. Regardless of the arrangement pattern of the color filters, for example, the column readout circuit can be modified so as to sequentially read out only the G pixels via the common terminal.

In the embodiment, a data processing method including the following steps (i) to (iv) is disclosed.
(i) In a case where data of each pixel is read out via a plurality of input terminals of the ADC through the read lines of the CMOS image sensor 14 for the OB region of the pixel array 141, data of each pixel of the pixel array 141 is read out in the shift readout, and FPN (an example of reference data) corresponding to each input terminal is acquired based on the data of the pixel read out via each input terminal;
(ii) In a case where data of each pixel is read out via a plurality of input terminals of the ADC through the readout lines of the CMOS image sensor 14 for the OB region of the pixel array 141, data of each pixel of the pixel array 141 is read out in the normal readout, and FPN (an example of reference data) corresponding to each input terminal is acquired based on the data of the pixel read out via each input terminal;
(iii) A readout mode is set based on FPN acquired in each of the shift readout and normal readout; and
(iv) Data of each pixel in the effective pixel area is corrected by subtracting the corresponding FPN from the data of the pixel read out via each input terminal in the set readout mode for the effective pixel region of the pixel array 141

In the embodiment, a program for causing a computer to execute the procedures (i) to (iv) is disclosed.

The imaging device, the electronic endoscope system, the data processing method, and the program according to the present invention have been described in detail above, but it is not limited to the above-described embodiment, and may of course be modified or altered in various manners in a range not deviating from the gist of the present invention.

### Reference Signs List

The present invention relates to a patent application of Japanese Patent Application No. 2023-113557 filed with the Japan Patent Office on July 11, 2023, the entire contents of which are incorporated herein by reference.

## Claims

1. An imaging device comprising:
an image sensor in which a color filter of either R (red), G (green), or B (blue) is disposed in a predetermined pattern for each pixel of a plurality of pixels arranged in a matrix and which is configured such that adjacent pixel columns share a readout line;
a readout unit that reads out data of each pixel via a plurality of terminals through the readout lines of the image sensor, the readout unit sequentially reading out data of pixels (G pixels) corresponding to G color filters in the adjacent pixel columns via a common terminal, and sequentially reading out data of pixels (R pixels) corresponding to R color filters and data of pixels (B pixels) corresponding to B color filters in the adjacent pixel columns via the common terminal;
an acquisition unit that acquires, for light-shielded pixel regions set in a plurality of pixel rows in the image sensor, reference data corresponding to each terminal based on data of pixels read out via the same terminal; and
a correction unit that corrects data of each pixel in an effective pixel region by subtracting corresponding reference data from data of pixels read out via each terminal for the effective pixel region excluding the light-shielded pixel regions in the image sensor.

2. An imaging device comprising:
an image sensor in which a color filter of either R (red), G (green), or B (blue) is disposed in a predetermined pattern for each pixel of a plurality of pixels arranged in a matrix and which is configured such that adjacent pixel columns share a readout line;
a readout unit that reads out data of each pixel via a plurality of terminals through the readout lines of the image sensor, the readout unit reading out the data of each pixel in the image sensor in either a first readout mode in which data of pixels (G pixels) corresponding to G color filters in the adjacent pixel columns are sequentially read out via a common terminal, and data of pixels (R pixels) corresponding to R color filters and data of pixels (B pixels) corresponding to B color filters in the adjacent pixel columns are sequentially read out via the common terminal, or
a second readout mode in which the data of the G pixels and the data of the R pixels in the adjacent pixel columns are sequentially read out via the common terminal and the data of the G pixels and the data of the B pixels in the adjacent pixel columns are sequentially read out via the common terminal;
an acquisition unit that acquires, for light-shielded pixel regions set in a plurality of pixel rows in the image sensor, reference data corresponding to each terminal based on data of pixels read out via each terminal;
a control unit that sets a readout mode when the readout unit reads out data of each pixel in the image sensor based on the reference data acquired by the acquisition unit in either the first readout mode or the second readout mode; and
a correction unit that corrects data of each pixel in an effective pixel region by subtracting corresponding reference data from data of pixels read out via each terminal in the readout mode set by the control unit for the effective pixel region excluding the light-shielded pixel regions in the image sensor.

3. The imaging device according to claim 2,
wherein the readout unit includes an analog-to-digital conversion unit including a plurality of analog-to-digital converters that convert, into digital data, data read out along pixel columns of the image sensor via a predetermined number of terminals among the plurality of terminals, and
each of the analog-to-digital converters is configured to sequentially process the data read out via the predetermined number of terminals.

4. An electronic endoscope system comprising:
the imaging device for imaging biological tissue according to claim 2; and
a light source device that emits illumination light to the biological tissue,
wherein the light source device is capable of selecting either white light or special light as the illumination light, and
the control unit sets a readout mode when the readout unit reads out data of each pixel in the image sensor at a time of activation or at a timing when the illumination light is selected.

5. The electronic endoscope system according to claim 4,
wherein in a case where the reference data acquired in either the first readout mode or the second readout mode is less than a predetermined level, the control unit sets the mode to a readout mode when the readout unit reads out data of each pixel in the image sensor.

6. The electronic endoscope system according to claim 4 or 5,
wherein in a case where the reference data acquired in either the first readout mode or the second readout mode is equal to or greater than a predetermined level, the control unit compares the reference data with reference data acquired in the other mode, and sets the readout mode corresponding to smaller reference data to a readout mode when the readout unit reads out data of each pixel in the image sensor.

7. A data processing method in a case where an object is imaged using an image sensor in which a color filter of either R (red), G (green), or B (blue) is disposed in a predetermined pattern for each pixel of a plurality of pixels arranged in a matrix and which is configured such that adjacent pixel columns share a readout line, the method comprising:
a step of reading out data of each pixel in the image sensor in a first readout mode in which data of pixels (G pixels) corresponding to G color filters in the adjacent pixel columns are sequentially read out via a common terminal and data of pixels (R pixels) corresponding to R color filters and data of pixels (B pixels) corresponding to B color filters in the adjacent pixel columns are sequentially read out via the common terminal in a case where data of each pixel is read out via a plurality of terminals through the readout lines of the image sensor for light-shielded pixel regions set in a plurality of pixel rows in the image sensor, and acquiring reference data corresponding to each terminal based on the data of pixels read out via each terminal;
a step of reading out data of each pixel in the image sensor in a second readout mode in which the data of the G pixels and the data of the R pixels in the adjacent pixel columns are sequentially read out via the common terminal and the data of the G pixels and the data of the B pixels in the adjacent pixel columns are sequentially read out via the common terminal in a case where the data of each pixel is read out via the plurality of terminals through the readout lines of the image sensor for the light-shielded pixel regions, and acquiring reference data corresponding to each terminal based on the data of pixels read out via each terminal;
a step of setting a readout mode based on the reference data acquired in either the first readout mode or the second readout mode; and
a step of correcting data of each pixel in an effective pixel region by subtracting corresponding reference data from data of pixels read out via each terminal in the set readout mode for the effective pixel region excluding the light-shielded pixel regions in the image sensor.

8. A program for causing a computer to execute a procedure in a case where an object is imaged using an image sensor in which a color filter of either R (red), G (green), or B (blue) is disposed in a predetermined pattern for each pixel of a plurality of pixels arranged in a matrix and which is configured such that adjacent pixel columns share a readout line, the procedure comprising:
a step of reading out data of each pixel in the image sensor in a first readout mode in which data of pixels (G pixels) corresponding to G color filters in the adjacent pixel columns are sequentially read out via a common terminal and data of pixels (R pixels) corresponding to R color filters and data of pixels (B pixels) corresponding to B color filters in the adjacent pixel columns are sequentially read out via the common terminal in a case where data of each pixel is read out via a plurality of terminals through the readout lines of the image sensor for light-shielded pixel regions set in a plurality of pixel rows in the image sensor, and acquiring reference data corresponding to each terminal based on the data of pixels read out via each terminal;
a step of reading out data of each pixel in the image sensor in a second readout mode in which the data of the G pixels and the data of the R pixels in the adjacent pixel columns are sequentially read out via the common terminal and the data of the G pixels and the data of the B pixels in the adjacent pixel columns are sequentially read out via the common terminal in a case where the data of each pixel is read out via the plurality of terminals through the readout lines of the image sensor for the light-shielded pixel regions, and acquiring reference data corresponding to each terminal based on the data of pixels read out via each terminal;
a step of setting a readout mode based on the reference data acquired in either the first readout mode or the second readout mode; and
a step of correcting data of each pixel in an effective pixel region by subtracting corresponding reference data from data of pixels read out via each terminal in the set readout mode for the effective pixel region excluding the light-shielded pixel regions in the image sensor.
